# EUROPEAN PATENT APPLICATION

(11) **EP 3 973 999 A1**
(43) Date of publication of application: **30.03.2022**
(21) Application number: 20306109.8
(22) Date of filing: 28.09.2020
(51) Int. Cl.: A61K 51/08, A61P 35/00

(54) **CONJUGATE OR ITS SALT COMPRISING A GASTRIN-RELEASING PEPTIDE RECEPTOR ANTAGONIST AND USES THEREOF**

(71) Applicant: Orano Med, 92320 Chatillon (FR)
(72) Inventor: TORGUE, Julien, Gaithersburg, MD 20878 (US); STALLONS, Tania, Wylie, TX 75098 (US); WONG, Amy, Richardson, TX 75081 (US); SAIDI, Amal, 74200 Thonons-Les-Bains (FR)
(74) Representative: Brevalex

(57) **Abstract**

The invention relates to a conjugate or a pharmaceutically acceptable salt thereof, the conjugate being of formula: C-L-A, wherein C is a chelator, L is a linker covalently bound to the chelator and A is a Gastrin-releasing peptide receptor antagonist covalently bound to the linker, characterized in that:
- the chelator is of formula: 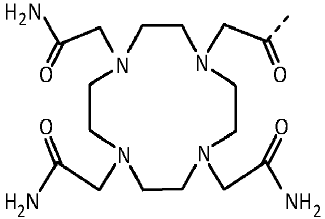 where the dotted line represents the covalent bond to the linker;
- the linker is of formula: -β-Ala-β-Ala-; and
- the Gastrin-releasing peptide receptor antagonist has the amino acid sequence: -DPhe-Gln-Trp-Ala-Val-Gly-His-Sta-Leu-NH₂.

The invention also relates to the uses of the conjugate or salt thereof.

## Description

### TECHNICAL FIELD

The invention belongs to the field of radiopharmaceuticals.

More specifically, the invention relates to a conjugate or a pharmaceutically acceptable salt thereof, which comprises a Gastrin-releasing peptide receptor (GRPR) antagonist and which may be used either for preparing a radiopharmaceutical or, once labelled with a radionuclide, as a radiopharmaceutical.

The invention also relates to a composition, a radiopharmaceutical as well as a kit-of-parts comprising the conjugate or the salt thereof.

The invention further relates to the use of the unlabelled conjugate or the salt thereof as well as of the kit-of-parts for preparing a radiopharmaceutical.

The invention still relates to the radiopharmaceutical for use in the *in vivo* imaging or the treatment of cancers in which the GRPR is overexpressed and, more particularly, prostate, breast and lung cancers.

### BACKGROUND OF THE INVENTION

Prostate cancer is the most common cancer among men, except for skin cancer and the second leading cause of cancer death in men in the United States.

Several treatment options are presently proposed to prostate cancer patients depending on the type of cancer cells and the stage of development of the cancer, the age and the general health of the patients, such as active surveillance, surgery, external radiotherapy, cryotherapy, hormone therapy, high-intensity focused ultrasounds and chemotherapy.

Nevertheless, there is a strong need for improved therapy.

One promising way of improved therapy for prostate cancer is the use of targeted radiopharmaceuticals, that is to say of drugs which are labelled with a radionuclide and which are able to target the cancer cells so as to deliver a toxic level of radiation to the cancer cells whilst sparing normal healthy tissues.

Typically, radiopharmaceuticals designed to prostate cancer are conjugates comprising a vector molecule with high affinity for prostate cancer cells and which is linked to, possibly *via* a linker (or spacer), a chelator in which the radionuclide is retained by chelation.

The GRPR, also known as bombesin (BBN) receptor subtype II, has been shown to be overexpressed in several human tumors, including prostate tumors but also breast and lung tumors. Overexpression of GRPR was found in 63 %-100 % of primary prostate cancers and more than 50 % of lymph and bone metastases. The GRPR density was reported to be 26-fold higher in prostatic carcinoma than prostatic hyperplasia.

Therefore, a variety of conjugates has been proposed for targeting GRPR-positive tumors and notably prostate cancers.

Recent reports have shown that GRPR antagonists have properties superior to conjugates GRPR agonists, affording higher tumor uptake and lower accumulation in physiologic GRPR-positive non target tissues. Moreover, GRPR agonists were shown to induce side effects in patients, mediated by virtue of their physiologic activity.

Therefore, particular attention has been drawn to the development of conjugates comprising a GRPR antagonist rather than a GRPR agonist as a vector molecule.

Examples of such conjugates are, for example, disclosed in the European patent application No. 2 252 628.

However, contrary to what the teaching of this reference might lead to think, the design of a GRPR antagonist-based conjugate able to be really used as a targeted radiopharmaceutical for treating GRPR-positive tumors is a major challenge because the pharmacokinetic and tumor targeting properties of a GRPR antagonist-based conjugate cumulatively depend on the choice of the chelator, the choice of the linker and the choice of the GRPR antagonist.

### SUMMARY OF THE INVENTION

The invention sets out precisely to propose a conjugate which results in an unexpected high and persistent uptake in GRPR-positive tumors such as prostate tumors combined with a low uptake and rapid clearance in non-target organs, as well as a pharmaceutically acceptable salt thereof.

The conjugate meets formula: C-L-A, wherein C is a chelator, L is a linker covalently bound to the chelator and A is a GRPR antagonist covalently bound to the linker, and is characterized in that:
- the chelator corresponds to the chelator known as DOTAM (1,4,7,10-tetrakis(carbamoylmethyl)-1,4,7,10-tetraazacyclododecane) and is of formula: 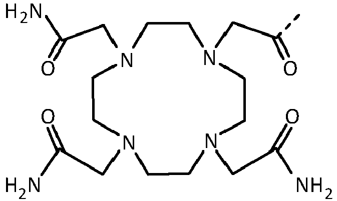 where the dotted line represents the covalent bond to the linker;
- the linker is of formula: -β-Ala-β-Ala-; and
- the GRPR antagonist is the peptide known as JMV 594, of amino acid sequence: -DPhe-Gln-Trp-Ala-Val-Gly-His-Sta-Leu-NH₂ (SEQ ID NO: 1).

In other words, the conjugate replies to formula:

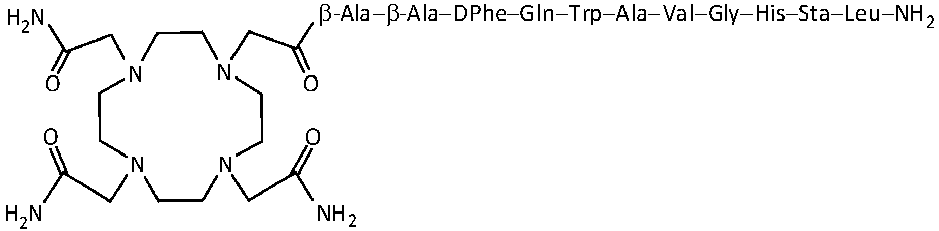

In what precedes and what follows:
- β-Ala refers to the beta-alanine, also known as 3-aminopropanoic acid;
- DPhe, Gln, Trp, Ala, Val, Gly, His and Leu refer to the α-amino acids phenylalanine, glutamine, tryptophan, alanine, valine, glycine, histidine and leucine respectively, the phenylalanine being in D-form whilst the glutamine, tryptophan, alanine, valine, histidine and leucine are in L-form; whereas
- Sta refers to the γ-amino acid statine of formula: 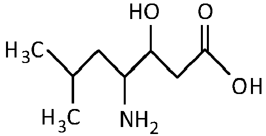 also known as (35,45)-4-amino-3-hydroxy-6-methylheptanoic acid.

Furthermore, the term "pharmaceutically-acceptable salt" refers to salts which possess toxicity profiles within a range that affords utility in pharmaceutical applications.

Suitable pharmaceutically-acceptable may notably be addition salts of free acids or free bases.

Acid addition salts may be prepared from an inorganic acid or from an organic acid. Appropriate inorganic acids include hydrochloric, hydrobromic, hydriodic, nitric, carbonic, sulfuric, and phosphoric acids, whereas appropriate organic acids may be selected from aliphatic, cycloaliphatic, aromatic, araliphatic, heterocyclic, carboxylic and sulfonic organic acids, examples of which include formic, acetic, propionic, succinic, glycolic, gluconic, lactic, malic, tartaric, citric, ascorbic, glucuronic, maleic, fumaric, pyruvic, aspartic, glutamic, benzoic, anthranilic, 4-hydroxybenzoic, phenylacetic, mandelic, embonic (pamoic), methanesulfonic, ethanesulfonic, benzenesulfonic, pantothenic, trifluoromethanesulfonic, 2-hydroxyethanesulfonic, p-toluenesulfonic, sulfanilic, cyclohexylaminosulfonic, stearic, alginic, β-hydroxybutyric, salicylic, galactaric and galacturonic acid.

Base addition salts are, for example, metallic salts including alkali metal, alkaline earth metal and transition metal salts such as, for example, calcium, magnesium, potassium, sodium and zinc salts, or organic salts made from basic amines such as, for example, *N,N*-dibenzylethylenediamine, chloroprocaine, choline, diethanolamine, ethylenediamine, meglumine (N-methylglucamine) and procaine.

For use as a radiopharmaceutical, the conjugate or the salt thereof further comprises a radionuclide chelated by the chelator.

The invention also relates to a composition which comprises the conjugate or the salt thereof in unlabelled form (i.e. devoid of any radionuclide) in a pharmaceutically acceptable medium such as saline, metal-free water, ascorbic acid, ethanol, polysorbate 80 (i.e. polyoxyethylene (20) sorbitan monooleate, sold under the trademark Tween^{™} 80), a buffer such as an ammonium acetate buffer, or a mixture thereof, ascorbic acid and ethanol acting advantageously as antioxidants whereas polysorbate 80 reduces advantageously stickiness.

The invention further relates to a radiopharmaceutical ready for use, which comprises the conjugate or the salt thereof in radiolabelled form (i.e. comprising the radionuclide chelated by the chelator) in a pharmaceutically acceptable medium such as mentioned above.

The invention also relates to a kit-of-parts which may be used for preparing a radiopharmaceutical and which comprises at least:
- a first container containing the conjugate or the salt thereof in unlabelled form; and
- a second container containing the radionuclide, typically in the form of a salt (chloride, acetate, ...).

In the kits-of-parts, the conjugate or the salt thereof and the radionuclide may be in any appropriate form, such as in dry form (powder for example), a liquid form, i.e. in solution in a pharmaceutically acceptable medium such as mentioned above, or in a frozen form.

As known *per se,* the kit may further comprise:
- one or more reagents and/or one or more solvents or diluents such as saline, metal-free water, biological buffer and the like, and/or
- a booklet with instructions for preparing and/or using the radiopharmaceutical.

The invention further relates to the use of the unlabelled conjugate, the salt thereof or the kit-of-parts, for preparing a radiopharmaceutical, which use comprises a chelation of the radionuclide by the chelator of the conjugate or salt thereof.

In what precedes, the radionuclide is preferably a lead radionuclide, in particular ²⁰³Pb if the radiopharmaceutical is intended to be used for *in vivo* imaging purposes or ²¹²Pb if the radiopharmaceutical is intended to be used for therapy purposes.

The invention still relates to the radiopharmaceutical for use in the *in vivo* imaging, for example by Single-Photon Emission Computed Tomography (SPECT), or the treatment of a cancer in which the Gastrin-releasing peptide receptor is overexpressed.

Such a use comprises administering an appropriate dose of the radiopharmaceutical to the patient to be imaged or treated, typically intravenously, and, in case of an *in vivo* imaging, subjecting the patient to the imaging.

Preferably, the cancer is a prostate, breast or lung cancer, with or without metastases, in particular a prostate cancer.

Other characteristics and advantages of the invention will become better apparent on reading the complement to the description that follows.

Obviously, this complement to the description is only given to illustrate the object of the invention and does not constitute in any case a limitation of said object.

### BRIEF DESCRIPTION OF THE FIGURES

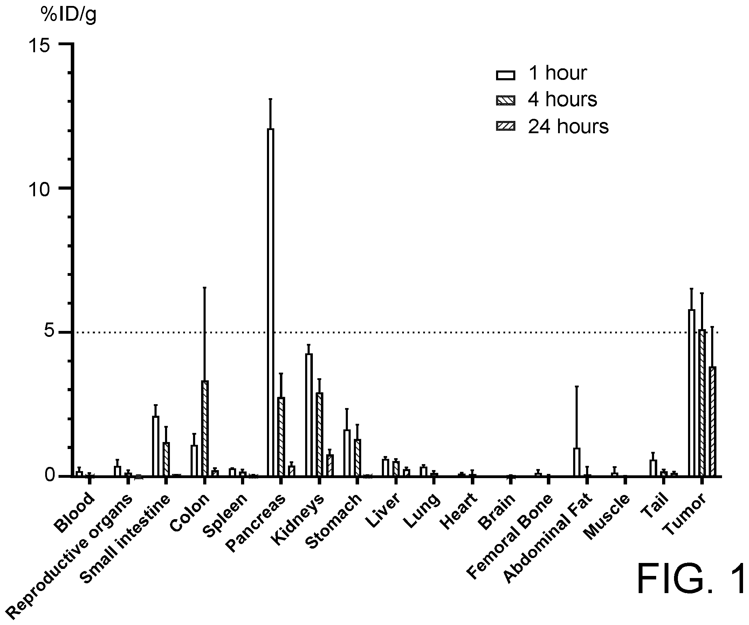

Figure 1 illustrates the results of a biodistribution study made with the conjugate of the invention, labelled with ²¹²Pb at a specific activity of 10 µCi per 14 ng, in athymic nude mice bearing subcutaneous PC-3 tumors; the results are expressed in terms of percent injected dose per gram of organ, noted as %ID/g, as found in the organs of the mice at 1 hour, 4 hours and 24 hours after injection of the ²¹²Pb-conjugate doses in the mice.

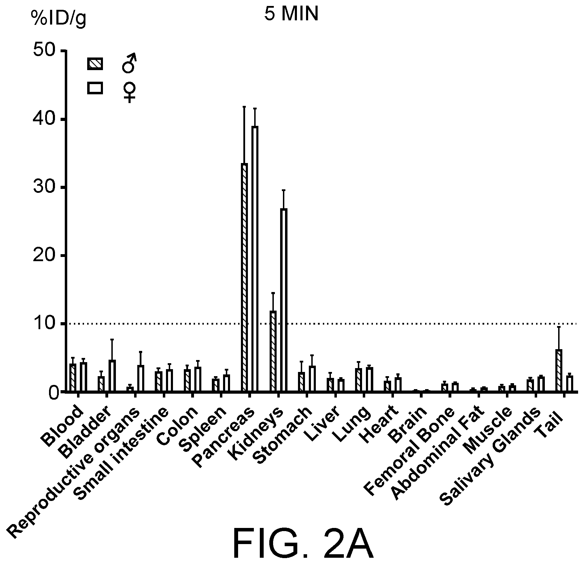

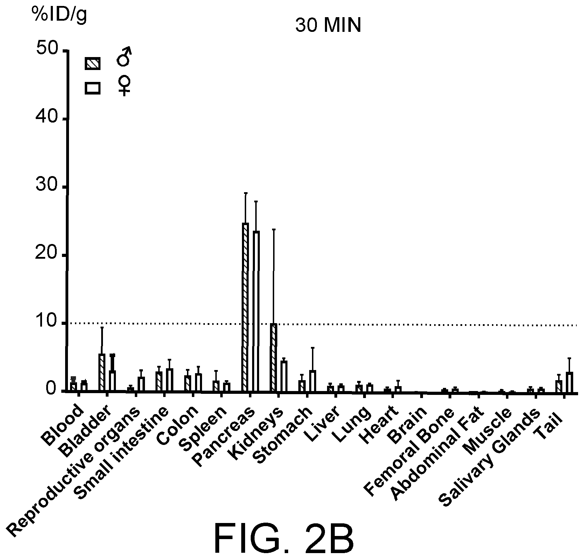

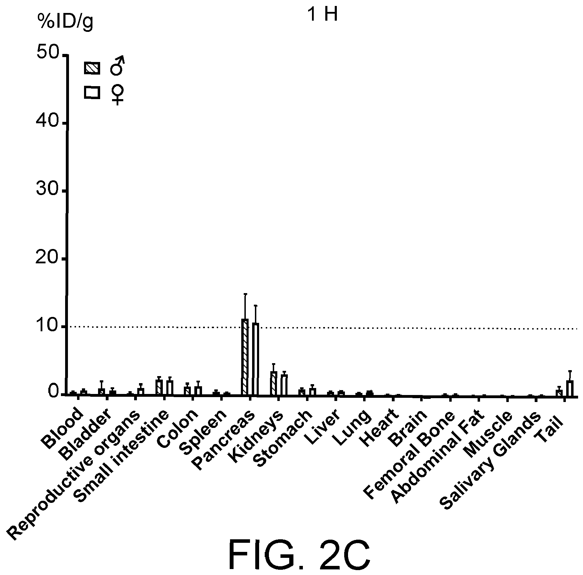

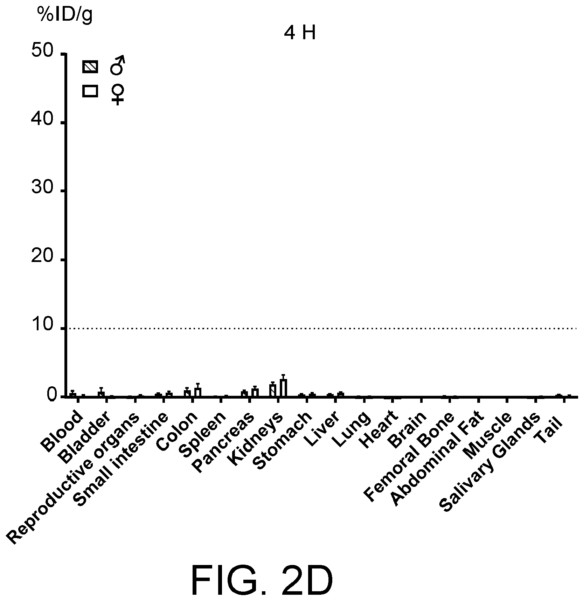

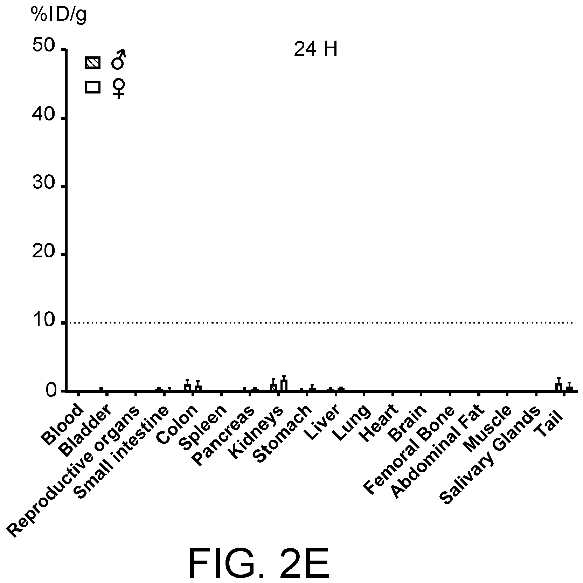

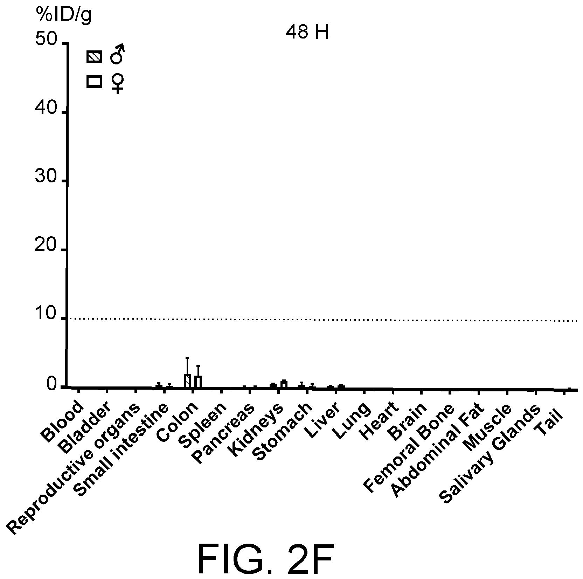

Figures 2A to 2F illustrate the results of a biodistribution study made with the conjugate of the invention, labelled with ²⁰³Pb at a specific activity of 10 µCi per 28 ng, in tumor-free immunocompetent mice; the results are expressed in terms of percent injected dose per gram of organ, noted as %ID/g, as found in the organs of the mice at 5 min (figure 2A), 30 min (figure 2B), 1 hour (figure 2C), 4 hours (figure 2D), 24 hours (figure 2E) and 48 hours (figure 2F) after injection of the ²⁰³Pb-conjugate doses in the mice.

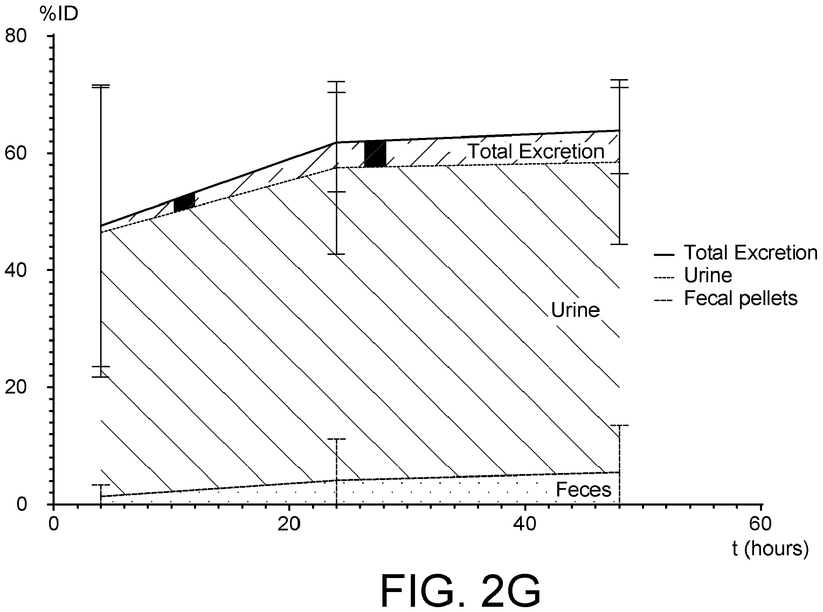

Figure 2G illustrates the urinary, fecal and total excretions of the conjugate of the invention, labelled with ²⁰³Pb, expressed in terms of percent injected dose, noted as %ID, in tumor-free immunocompetent mice as a function of time after injection of the ²⁰³Pb-conjugate doses in the mice, noted as t and expressed in hours.

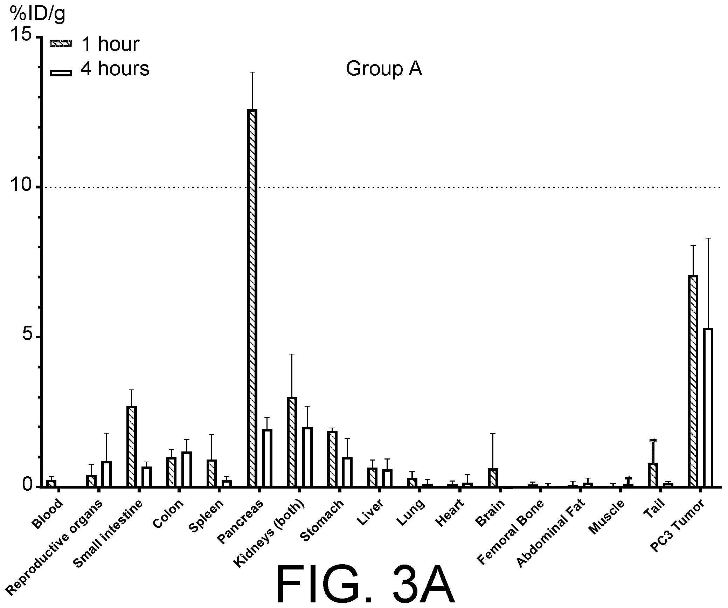

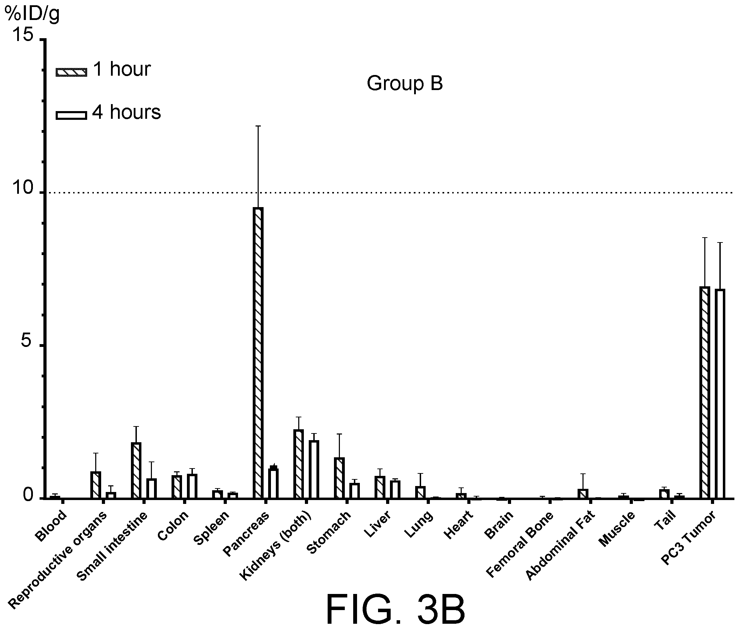

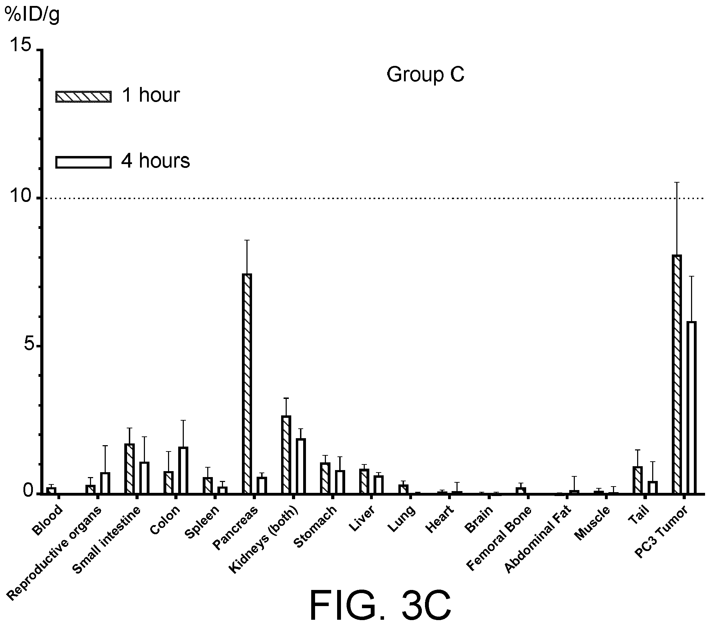

Figures 3A to 3C illustrate the results of a biodistribution study made with the conjugate of the invention, labelled with ²¹²Pb at different specific activities, in athymic nude mice bearing subcutaneous PC-3 tumors; figure 3A corresponds to a first group of mice, denoted as group A, having received one ²¹²Pb-conjugate dose of specific activity equal to 10 µCi per 28 ng; figure 3B corresponds to a second group of mice, denoted as group B, having received one ²¹²Pb-conjugate dose of specific activity equal to 10 µCi per 140 ng whilst figure 3C corresponds to a third group of mice, denoted as group C, having received one ²¹²Pb-conjugate dose of specific activity equal to 10 µCi per 280 ng; in each figure, the results are expressed in terms of percent injected dose per gram of organ, noted as %ID/g, as found in the organs of the mice at 1 hour and 4 hours after injection of the ²¹²Pb-conjugate doses in the mice.

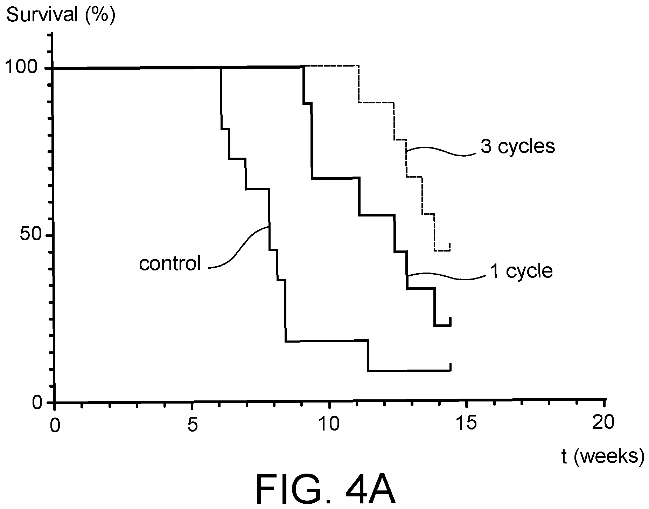

Figure 4A illustrates the survival, expressed in %, of athymic nude mice bearing subcutaneous PC-3 tumors and having received either only one dose of the conjugate of the invention, labelled with ²¹²Pb at a specific activity of 10 µCi per 14 ng (1 cycle), or three doses of the same conjugate at intervals of 14 days (3 cycles), or sterile saline (control), as a function of time after injection of the cancer cells in the mice, noted as t and expressed in weeks.

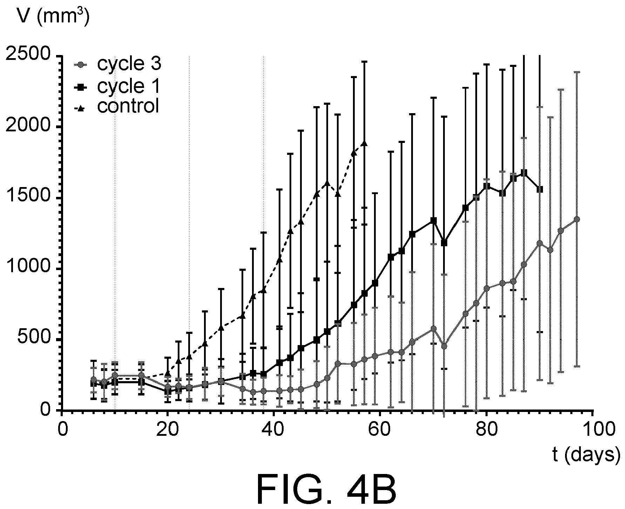

Figure 4B illustrates the average tumor volume, noted as V and expressed in mm³, presented by athymic nude mice bearing subcutaneous PC-3 tumors and having received either only one dose of the conjugate of the invention, labelled with ²¹²Pb at a specific activity of 10 µCi per 14 ng (1 cycle), or three doses of the same conjugate at intervals of 14 days (3 cycles), or sterile saline (control), as a function of time after injection of the cancer cells in the mice, noted as t and expressed in weeks.

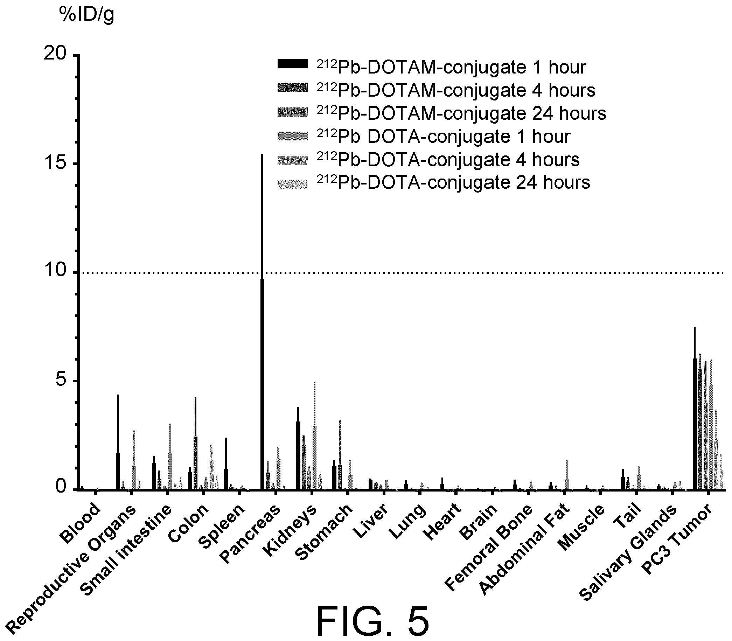

Figure 5 illustrates the results of a comparative study aimed at comparing the biodistribution of the conjugate of the invention, labelled with ²¹²Pb at a specific activity of 10 µCi per 280 ng, with that of a conjugate, also labelled with ²¹²Pb at the same specific activity, only differing from the conjugate of the invention in that it comprises DOTA as a chelator, in athymic nude mice bearing subcutaneous PC-3 tumors; the results are expressed in terms of percent injected dose per gram of organ, noted as %ID/g, as found in the organs of the mice at 1 hour, 4 hours and 24 hours after injection of the ²¹²Pb-conjugate doses in the mice; in this figure, the conjugate of the invention is denoted as ²¹²Pb-DOTAM-conjugate whilst the comparative conjugate is denoted as ²¹²Pb-DOTA-conjugate.

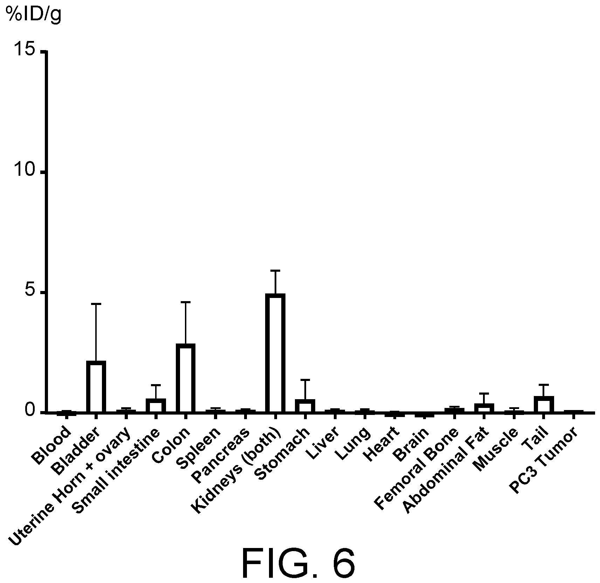

Figure 6 illustrates the results of a comparative study aimed at assessing the biodistribution of a conjugate, labelled with ²¹²Pb at a specific activity of 10 µCi per 10 ng, only differing from the conjugate of the invention in that it comprises a linker constituted by a chain of 3 glutamic acid residues, in athymic nude mice bearing subcutaneous PC-3 tumors; the results are expressed in terms of percent injected dose per gram of organ, noted as %ID/g, as found in the organs of the mice at 4 hours after injection of the conjugate doses in the mice.

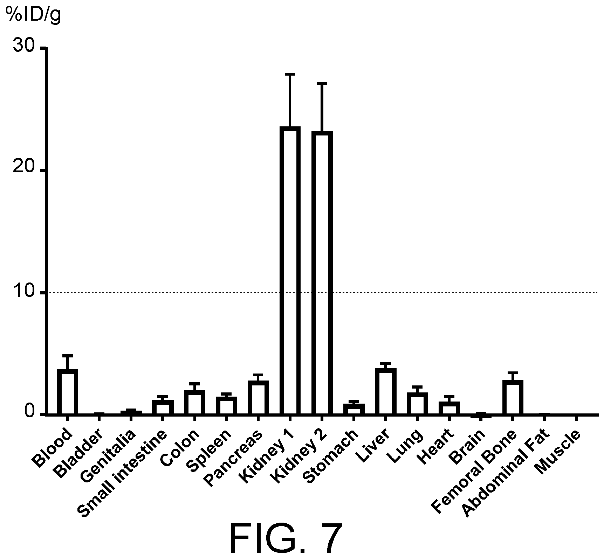

Figure 7 illustrates the results of a comparative study aimed at assessing the biodistribution of a conjugate, labelled with ²¹²Pb at a specific activity of 10 µCi per 4.1 ng, only differing from the conjugate of the invention in that it comprises a linker constituted by a 4-amino-(1-carboxymethyl)piperidinyl group, in tumor-free immunocompetent mice; the results are expressed in terms of percent injected dose per gram of organ, noted as %ID/g, as found in the organs of the mice at 4 hours after injection of the conjugate doses in the mice.

### DETAILED DESCRIPTION OF THE INVENTION

### I - PREPARATION OF THE UNLABELLED CONJUGATE OF THE INVENTION:

### 1.1- Preparation of the peptide sequence β-Ala-β-Ala-DPhe-Gln-Trp-Ala-Val-Gly-His-Sta-Leu-NH₂ (SEQ ID NO: 2):

An automated microwave peptide synthesizer (Biotage^{™} Initiator + Alstra^{™} - BIOTAGE^{™}) was used for the synthesis of the peptide sequence: β-Ala-β-Ala-DPhe-Gln-Trp-Ala-Val-Gly-His-Sta-Leu-NH₂) on a 0.1 mmol scale.

Standard 9-fluorenylmethoxycarbonyl (Fmoc) chemistry was used with 2-(1*H-*benzotriazol-1-yl)-1,1,3,3-tetramethyluronium hexafluorophosphate (HBTU) and *N*-hydroxylbenzotriazole (HOBt) as activators.

A rink amide resin was used to provide an amidated C-terminus.

The amino acids leucine, valine and β-alanine were double coupled. In addition to being double coupled, the two β-alanine were double deprotected.

All the amino acids were coupled at 75 °C with the exception of histidine and statine which were coupled at 48 °C to avoid a racemization of the histidine and an O-acylation of the statine.

### I.2 - Conjugation of the DOTAM to the peptide sequence:

The DOTAM was conjugated to the peptide sequence bound to the resin by using the DOTAM monoacid of formula:

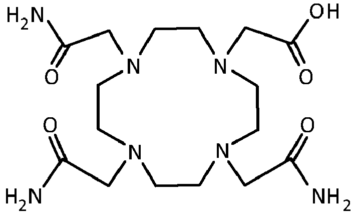

To do this, the DOTAM monoacid was firstly preactivated by dissolving, in a round bottom flask, 2.25 equivalents of DOTAM monoacid (0.225 mmol; 90.5 mg), 2.25 equivalents of 1-[bis(dimethylamino)methylene]-1H-1,2,3-triazolo[4,5,-b]pyridinium 3-oxide hexafluorophosphate (HATU; 0.225 mmol; 85.5 mg) and 6.75 equivalents of diisopropylethylamine (DIEA; 0.675 mmol; 120 µL) in 3 mL of dimethylformamide (DMF) and stirring the mixture for 30 min.

Then, the peptide sequence bound to the resin was added to the mixture and the reaction was allowed to spin overnight.

Following completion of the conjugation, the reaction medium was filtered over a course fritted funnel to remove excess reagents and the residue was washed three times with DMF, three times with methanol and three times with DCM.

### I.3 - Cleavage of the conjugate from the resin:

The conjugate was cleaved from the resin by suspending in a cocktail composed of 95 % (v/v) trifluoroacetic acid (TFA), 2.5 % (v/v) triisopropylsilane (TIPS) and 2.5 % (v/v) H₂O to a final volume of 3 mL.

The reaction was spun in a round bottom flask for three hours after which the reaction medium was filtered over a course fritted funnel. The TFA was evaporated using nitrogen gas and the conjugate was precipitated using cold ethyl ether. The flask was then submitted to a centrifugation at 4500 rpm for 10 min and the ethyl ether supernatant was removed. The pellet was then freeze dried overnight to remove the excess of ethyl ether.

### I.4 - Purification of the conjugate:

The conjugate was purified by means of reverse-phase HPLC using a PHENOMENEX^{™} Luna^{™} 10 µm C18(2) preparative column (250 x 50 mm) with as a gradient:
- t = 0-5 min: eluent A (0.1 % TFA in water) comprising 1 % eluent B (0.1 % TFA in acetonitrile (ACN));
- t = 5-45 min: eluent B rising linearly from 1 % to 75 % in eluent A.

The pure conjugate had a retention time of ≈ 24 min. The collected peak was submitted to a rotary evaporation to remove the organic solvent and freeze dried.

It was thus obtained 13 mg of the conjugate with a purity > 95 % as determined with a AGILENT^{™} 1100 Series LC-MS using a RESTREK^{™} Ultra IBD 3 µm analytical column (150 x 2.1 mm) with as a gradient:
- t = 0-2 min: eluent B (100 % H₂O);
- t = 2-17 min: eluent B decreasing linearly from 100 % to 0 % in eluent A (0.1 % TFA in ACN).

The mass of the pure peptide was confirmed with the AGILENT^{™} 1100 Series LC-MS coupled with a HEWLETT PACKARD^{™} 1100 Series MSD: expected 1638.91; observed 1638.7.

The conjugate was stored at -80 °C for later lead labelling.

### II - RADIOLABELLING OF THE CONJUGATE OF THE INVENTION:

For *in vivo* distribution and efficacy studies in mice, conjugates labelled with ²¹²Pb or ²⁰³Pb, denoted hereinafter as "²¹²Pb-conjugate" and "²⁰³Pb-conjugate" respectively, were prepared on the day of injection to the mice, based on the specific activity at the time of conjugation and diluted for the particular activity needed at the time of injection.

For doing that, the conjugate as obtained under item I above was thawed and diluted in metal free water. Then, an appropriate volume of the so obtained conjugate solution was added to a cryogenic vial possibly containing appropriate volumes of 0.4 M ammonium acetate, ascorbic acid, ethanol and tween solutions. This was followed by an appropriate volume of a ²¹²Pb-acetate solution (ORANO MED) or ²⁰³Pb-chloride solution (LANTHEUS) that may have been pH adjusted with NaOH/0.4M ammonium acetate solution.

The samples were incubated at 50°C for 10 min and the chelation of ²¹²Pb or ²⁰³Pb by the conjugates was verified by measuring the ²¹²Pb or ²⁰³Pb remained free in the samples using instant thin layer chromatography (iTLC).

### III - IN VIVO STUDIES WITH LEAD LABELLED CONJUGATES OF THE INVENTION:

In what follows:
   * the athymic nude mice used are Hsd:Athymic Nude-Foxn1^{nu} mice from ENVIGO^{™};
   * the immunocompetent mice used are Hsd:ICR (CD-1^{™}) mice from ENVIGO^{™};
   * the PC-3 human prostate cancer cells used are ATCC^{™} CRL-1435^{™} cells from ATCC^{™};
   * the automatic gamma counter used is the PERKIN ELMER^{™} Wizard^{2™} counter; while
   * "Buffer 1" refers to a mixture of saline, 23 mM ascorbic acid, 0.08 % (v/v) Tween^{™} 20 and 5 % (v/v) ethanol.
   * "Buffer 2" refers to a mixture of saline, 20mM ascorbic acid, 0.02 % (v/v) Tween^{™} 80 and 5% (v/v) ethanol.

### III.1 - ²¹²Pb-conjugate biodistribution study in xenograft-bearing mice:

The instant study was aimed at assessing the biodistribution of the conjugate labelled with ²¹²Pb at a specific activity of 10 µCi per 14 ng in athymic nude mice bearing a human prostate cancer cell tumor.

### * ²¹²Pb-labelling of the conjugate at 10 µCi per 14 ng of conjugate:

| **Solutions** | **Volumes** | **Free ²¹²Pb** |
|---|---|---|
| Conjugate (17 ng/µL) | 88.2 µL | <5% |
| ²¹²Pb (1.99 µCi/µL) | 753.8 µL | |

### * Preparation of ²¹² Pb-conjugate doses of ≈ 10 µCi/100 µL:

| **Solutions** | **Volumes** |
|---|---|
| ²¹²Pb-conjugate (0.507 µCi/µL) | 811.4 µL |
| Buffer 1 | 2 788.6 µL |

Insulin syringes each containing 100 µL of the solution resulting from the mixture ²¹²Pb-conjugate/buffer 1 and corresponding to one ²¹²Pb-conjugate dose of ≈ 10 µCi/ 100 µL were prepared for injection to the mice.

### * Study design:

15 male athymic nude mice, 7-8 weeks old and weighting 27.74 ± 1.87 g at study initiation, were injected subcutaneously, into the right flank, with 10⁶ PC-3 human prostate cancer cells in 100 µL of RPMI-1640 medium/Matrigel^{™} (v/v: 1/1). The tumors were let grow until they reached 200-300 mm³ (as determined by the formula: volume = 0.5 x length x width²).

Then each mouse received intravenously (into a tail vein) one ²¹²Pb-conjugate dose.

After that, the mice were divided into 3 groups of 5, denoted as "group A", "group B" and "group C" respectively.

The mice of group A were sacrificed at 1 hour post-dose injection; the mice of group B were sacrificed at 4 hours post-dose injection whereas the mice of group C were sacrificed at 24 hours post-dose injection.

Blood, reproductive organs, small intestine, colon with caecum, spleen, pancreas, kidneys, stomach, liver, lung, heart, brain, femoral bone, abdominal fat, skeletal muscle, tail (as injection site) and PC-3 tumor were collected from each sacrificed mouse, weighted and transferred to individual tubes for automatic gamma counter.

The tubes were counted for two min. A standard consisting of 5 µL of the solution injected to the mice was also counted for each group of mice. The background was automatically subtracted from the counts. The standard was also used for decay correction.

The percent injected dose per gram, noted as %ID/g, was calculated for each organ collected (mean ± standard deviation).

### * Results:

The results are illustrated in figure 1.

As shown by this figure, at 1 hour post-dose injection, the highest uptake (≈ 12 %ID/g) of the ²¹²Pb-conjugate is observed in the pancreas likely due to the well-known GRPR expression in the pancreas. However, the uptake of the ²¹²Pb-conjugate in the tumor is also high (≈ 6 %ID/g) and slightly decreases at 4 hours and 24 hours post-dose injection.

The ²¹²Pb-conjugate has a fast clearance which results in a high tumor/blood ratio.

### III.2 - ²⁰³Pb-conjugate biodistribution study in tumor-free immunocompetent mice:

The instant study was aimed at assessing the biodistribution of the conjugate labelled with ²⁰³Pb at a specific activity of 10 µCi per 28 ng in tumor-free immunocompetent mice.

### * ²⁰³Pb-labelling of the conjugate at 10 µCi per 28 ng of conjugate:

| **Solutions** | **Volumes** | **Free ²⁰³Pb** |
|---|---|---|
| Conjugate (1 mg/mL) | 8.4 µL | <5% |
| ²⁰³Pb (108.3 µCi/µL) | 24.6 µL | |
| Ammonium acetate (0.4 M) | 475.4 µL | |
| Ascorbic acid (500 mM) | 33.3 µL | |
| NaOH (1 M) | 2 µL | |

### * Preparation of ²⁰³ Pb-conjugate doses of ≈ 10 µCi/100 µL:

| **Solutions** | **Volumes** |
|---|---|
| ²⁰³Pb-conjugate (2.78 µCi/µL) | 356.7 µL |
| Buffer 1 | 8 643.3 µL |

Insulin syringes each containing 100 µL of the solution resulting from the mixture ²⁰³Pb-conjugate/buffer 1 and corresponding to one ²⁰³Pb-conjugate dose of ≈ 10 µCi/ 100 µL were prepared for injection to mice.

### * Study design:

30 male and 30 female immunocompetent CD1 mice, 7-8 weeks old and weighting 27.75 ± 2.52 g for the male and 25.91 ± 2.75 g for the female at study initiation, received intravenously one ²⁰³Pb-conjugate dose.

After that, the mice were divided in 6 groups of 10, denoted as groups A, B, C, D, E and F respectively, each comprising 5 male and 5 female.

The mice of group A were sacrificed at 5 min post-dose injection; the mice of group B were sacrificed at 30 min post-dose injection; the mice of group C were sacrificed at 1 hour post-dose injection; the mice of group D were sacrificed at 4 hours post-dose injection whereas the mice of group E were sacrificed at 24 hours post-dose injection.

The mice of group F were placed in metabolic cages and their urinary and fecal excretions were collected at 4 hours, 24 hours and 48 hours post-dose injection; they were sacrificed at 48 hours post-dose injection.

Blood, bladder, reproductive organs, small intestine, colon with caecum, spleen, pancreas, kidneys, stomach, liver, lung, heart, brain, femoral bone, abdominal fat, skeletal muscle, salivary glands and tail were collected from each sacrificed mouse, weighted and transferred to individual tubes for automatic gamma counter.

The tubes were counted for two min. A standard consisting of 5 µL of the solution injected to the mice was also counted for each group of mice. The background was automatically subtracted from the counts. The standard was also used for decay correction.

The excretions of the mice of group F were also counted.

The percent injected dose per gram, noted as %ID/g, was calculated for each organ collected (mean ± standard deviation) whilst the percent injected dose, noted as %ID, was calculated for the excretions of the mice of group F (mean ± standard deviation).

### * Results:

The results are illustrated in figures 2A to 2G.

As shown by figures 2A to 2F, the ²⁰³Pb-conjugate has a safe biodistribution profile in both male and female mice.

Indeed, there is an initial high uptake (> 30 %ID/g at 5 min post-dose injection) of the ²⁰³Pb-conjugate in the pancreas but the %ID/g is well below 10 for all organs only at 4 hours post-dose injection.

No significant differences of %ID/g are observed between the male and female mice except for the kidney uptakes which are higher at the 5 min time-point in the female mice, probably because the female mice have smaller kidneys than those of the male mice leading to higher %ID per gram of organ.

Furthermore, figure 2G shows that the ²⁰³Pb-conjugate is mainly eliminated by renal excretion.

### III.3 - ²¹²Pb-conjugate biodistribution study in xenograft-bearing mice at different specific activity:

The instant study was aimed at assessing the biodistribution of the conjugate labelled with ²¹²Pb at a specific activity varying from 10 µCi per 28 ng to 10 µCi per 280 ng in athymic nude mice bearing a human prostate cancer cell tumor.

### * ²¹²Pb-labelling of the conjugate at 10 µCi per 28 ng of conjugate:

| **Solutions** | **Volumes** | **Free ²¹²Pb** |
|---|---|---|
| Conjugate (17 ng/µL) | 82.4 µL | <5% |
| ²¹²Pb (2.04 µCi/µL) | 245 µL | |
| Ascorbic acid (500 mM) | 16.3 µL | |

### * ²¹²Pb-labelling of the conjugate at 10 µCi per 140 ng of conjugate:

| **Solutions** | **Volumes** | **Free ²¹²Pb** |
|---|---|---|
| Conjugate (1 mg/mL) | 7 µL | <5% |
| ²¹²Pb (2.04 µCi/µL) | 245 µL | |
| Ascorbic acid (500 mM) | 16.3 µL | |

### * ²¹²Pb-labelling of the conjugate at 10 µCi per 280 ng of conjugate:

| **Solutions** | **Volumes** | **Free ²¹²Pb** |
|---|---|---|
| Conjugate (1 mg/mL) | 14 µL | <5% |
| ²¹²Pb (2.04 µCi/µL) | 245 µL | |
| Ascorbic acid (500 mM) | 16.3 µL | |

### * Preparation of ²¹² Pb-conjugate doses of ≈ 10 µCi/100 µL:

| **Specific activity** | **Solutions** | **Volumes** |
|---|---|---|
| 10 µCi per 28 ng | ²¹²Pb-conjugate (1.32 µCi/µL) | 134.9 µL |
| | Buffer 1 | 1 365.1 µL |
| 10 µCi per 140 ng | ²¹²Pb-conjugate (1.56 µCi/µL) | 114.2 µL |
| | Buffer 1 | 1 385.8 µL |
| 10 µCi per 280 ng | ²¹²Pb-conjugate (1.65 µCi/µL) | 107.9 µL |
| | Buffer 1 | 1 392.1 µL |

Insulin syringes each containing 100 µL of one of the solutions resulting from the mixtures ²¹²Pb-conjugate/buffer 1 and corresponding to one ²¹²Pb-conjugate dose of ≈ 10 µCi/100 µL were prepared for injection to mice.

### * Study design:

30 male athymic nude mice, 7-8 weeks old and weighting 27.89 ± 2.27 g at study initiation, were injected subcutaneously, into the right flank, with 10⁶ PC-3 human prostate cancer cells in 100 µL of RPMI-1640 medium/Matrigel^{™} (v/v: 1/1). The tumors were let grow until they reached 200-300 mm³.

The mice were divided into three groups of 10, denoted as groups A, B and C respectively.

Each mouse of group A received intravenously one ²¹²Pb-conjugate dose of specific activity equal to 10 µCi per 28 ng; each mouse of group B received intravenously one ²¹²Pb-conjugate dose of specific activity equal to 10 µCi per 140 ng whilst each mouse of group C received intravenously one ²¹²Pb-conjugate dose of specific activity equal to 10 µCi per 280 ng.

5 mice of each of groups A, B and C were sacrificed at 1 hour post-dose injection whilst 5 mice of each of groups A, B and C were sacrificed at 4 hours post-dose injection.

Blood, reproductive organs, small intestine, colon with caecum, spleen, pancreas, kidneys, stomach, liver, lung, heart, brain, femoral bone, abdominal fat, skeletal muscle, tail and PC-3 tumor were collected from each sacrificed mouse, weighted and transferred to individual tubes for automatic gamma counter.

The tubes were counted for two min. Standards consisting of 5 µL of the solutions injected to the mice were also counted. The background was automatically subtracted from the counts. The standards were also used for decay correction.

The percent injected dose per gram, noted as %ID/g, was calculated for each organ collected (mean ± standard deviation).

### * Results:

The results are illustrated in figures 3A to 3C.

As shown by these figures, the lower the specific activity of the ²¹²Pb-conjugate, the lower the healthy organ uptake, without affecting however the tumor uptake.

### 111.4 - ²¹²Pb-conjugate efficacy study in xenograft-bearing mice:

The instant study was aimed at assessing the efficacy of one treatment cycle (cycle 1) or three treatment cycles (cycles 1, 2 and 3) using the conjugate of the invention, labelled with ²¹²Pb at a specific activity of 10 µCi per 14 ng, in athymic nude mice bearing a human prostate cancer cell tumor.

### * ²¹²Pb-labelling of the conjugate at 10 µCi per 14 nq of conjugate:

| **Cycles** | **Solutions** | **Volumes** | **Free ²¹²Pb** |
|---|---|---|---|
| Cycle 1 | Conjugate (17 ng/µL) | 44.12 µL | <5% |
| | ²¹²Pb (2.42 µCi/µL) | 310 µL | |
| | Ascorbic acid (500 mM) | 20.67 µL | |
| Cycle 2 | Conjugate (17 ng/µL) | 29.4 µL | <5% |
| | ²¹²Pb (2.77 µCi/µL) | 734.2 µL | |
| | Ascorbic acid (500 mM) | 48.95 µL | |
| Cycle 3 | Conjugate (17 ng/µL) | 58.8 µL | <5% |
| | ²¹²Pb (4.9 µCi/µL) | 204.1 µL | |
| | Ascorbic acid (500 mM) | 13.6 µL | |

### * Preparation of ²¹² Pb-conjugate doses of ≈ 10 µCi/100 µL:

| **Cycles** | **Solutions** | **Volumes** |
|---|---|---|
| Cycle 1 | ²¹²Pb-conjugate (1.36 µCi/µL) | 334.6 µL |
| | Saline | 3 415.4 µL |
| Cycle 2 | ²¹²Pb-conjugate (0.354 µCi/µL) | 930.7 µL |
| | Saline | 499.3 µL |
| Cycle 3 | ²¹²Pb-conjugate (2.35 µCi/µL) | 1426.3 µL |
| | Saline | 73.7 µL |

Insulin syringes each containing 100 µL of one of the solutions resulting from the mixtures ²¹²Pb-conjugate/saline and corresponding to one ²¹²Pb-conjugate dose of ≈ 10 µCi/100 µL were prepared for injection to mice.

### * Study design:

40 male athymic nude mice, 7-8 weeks old and weighting 28.58 ± 1.97 g at study initiation, were injected subcutaneously, into the right flank, with 10⁶ PC-3 human prostate cancer cells in 100 µL of RPMI-1640 medium/Matrigel^{™} (v/v: 1/1). The tumors were let grow until they reached 200-300 mm³.

20 mice received intravenously one dose of cycle 1 at 10 days post-cancer cell injection whereas 10 mice received intravenously one dose of 100 µL of sterile saline (control).

10 of the 20 mice having received the dose of cycle 1 further received one dose of cycle 2 at 24 days post-cancer cell injection and one dose of cycle 3 at 38 days post-cancer cell injection.

During the study, the mice whose tumor volume reached 2 000 mm³ were euthanized immediately. Furthermore, the mice were euthanized before the scheduled endpoint when they showed signs of unamenable distress or pain due to tumor burden, side effects of the injections, or a combination of two or more of the following termination criteria: acute weight loss (e.g. 15 % weight loss over two consecutive days); poor tumor status (e.g. ulceration, teeth marks or open wounds); scruffiness/lack of grooming over 5 days; lethargy or reduced mobility over 3 days; weakness/balance issues over 5 days; hunchback appearance; diarrhea; paralysis; severe anemia and hypo-thermia).

### * Results:

The results are illustrated in figures 4A and 4B.

As shown by figure 4A, one or three treatment cycles using the conjugate of the invention lead to a mean survival time which is increased from 7.9 weeks (control) to 13.9 weeks (3 cycles).

There is no significant difference between one and three treatment cycles. It is likely that the time interval between two successive doses in the three treatment cycles is suboptimal and that the efficacy of a treatment with multiple doses may be increased by optimizing the time interval between two successive doses.

### IV - COMPARATIVE STUDIES:

### IV.1 - Impact of a change of chelator on the biodistribution in xenograft-bearing mice:

The instant study was aimed at comparing the biodistribution of the conjugate of the invention, labelled with ²¹²Pb, in athymic nude mice bearing a human prostate cancer cell tumor with that of a conjugate also labelled with ²¹²Pb and only differing from the conjugate of the invention in that the chelator corresponds to DOTA (1,4,7,10-tetraazacyclododecane-1,4,7,10-tetraacetic acid) and is of formula: 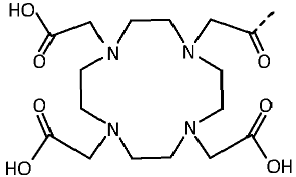 where the dotted line represents the covalent bond to the linker.

For sake of clarity, the conjugate of the invention is denoted hereinafter as "DOTAM-conjugate" whilst the comparative conjugate is denoted hereinafter as "DOTA-conjugate".

### * Preparation of the unlabelled DOTA-conjugate:

The unlabelled DOTA conjugate was prepared following the same protocol as described under item I above except that DOTAM was replaced with DOTA at the step of conjugation of the chelator to the peptide sequence.

### * ²¹²Pb-labelling of the DOTAM-conjugate and DOTA-conjugate at 10 µCi per 280 ng of conjugate:

| **Conjugates** | **Solutions** | **Volumes** | **Free ²¹²Pb** |
|---|---|---|---|
| DOTAM-conjugate | Conjugate (1 mg/µL) | 16.8 µL | < 5 % |
| | Ascorbic Acid (500 mM) | 8 µL | |
| | Absolute ethanol | 10 µL | |
| | Tween^{™} 80 | 0.04 µL | |
| | Metal Free Water | 28.8 µL | |
| | ²¹²Pb (4.4 µCi/µL) | 136.4 µL | |
| DOTA-conjugate | Conjugate (1 mg/µL) | 16.8 µL | < 5 % |
| | Ascorbic Acid (500 mM) | 8 µL | |
| | Absolute ethanol | 10 µL | |
| | Tween^{™} 80 | 0.04 µL | |
| | Metal Free Water | 28.8 µL | |
| | ²¹²Pb (4.4 µCi/µL) | 136.4 µL | |

### * Preparation of ²¹²Pb-DOTAM-conjugate and ²¹²Pb-DOTA-conjugate doses of ≈ 10 µCi/100 µL:

| **Conjugates** | **Solutions** | **Volumes** |
|---|---|---|
| ²¹²Pb-DOTAM-conjugate | ²¹²Pb-DOTAM-conjugate (2.73 µCi/µL) | 86.7 µL |
| | Buffer 2 | 1863.3 µL |
| ²¹²Pb-DOTA-conjugate | ²¹²Pb-DOTA-conjugate (3.14 µCi/µL) | 75.4 µL |
| | Buffer 2 | 1874.6 µL |

Insulin syringes each containing 100 µL of one of the solutions resulting from the mixtures ²¹²Pb-DOTAM-conjugate/buffer 2 and ²¹²Pb-DOTA-conjugate/buffer 2 and corresponding to one dose of ≈ 10 µCi/100 µL were prepared for injection to mice.

### * Study design:

30 male athymic nude mice, 7-8 weeks old and weighting 27.90 ± 1.9 g at study initiation, were injected subcutaneously, into the right flank, with 10⁶ PC-3 human prostate cancer cells in 100 µL of RPMI-1640 medium/Matrigel^{™} (v/v: 1/1). The tumors were let grow until they reached 200-300 mm³.

The mice were divided into 6 groups of 5, denoted as groups A, B, C, D, E and F respectively.

Each mouse of groups A, B and C received intravenously one ²¹²Pb-DOTAM-conjugate dose whilst each mouse of groups E, F and F received intravenously one ²¹²Pb-DOTA-conjugate dose.

The mice of groups A and D were sacrificed at 1 hour post-dose injection; the mice of groups B and E were sacrificed at 4 hour post-dose injection whilst the mice of groups C and F were sacrificed at 24 hours post-dose injection.

Blood, reproductive organs, small intestine, colon with caecum, spleen, pancreas, kidneys, stomach, liver, lung, heart, brain, femoral bone, abdominal fat, skeletal muscle, tail, salivary glands and PC-3 tumor were collected from each sacrificed mouse, weighted and transferred to individual tubes for automatic gamma counter.

The tubes were counted for two min. Standards consisting of 5 µL of the solutions injected to the mice were also counted. The background was automatically subtracted from the counts. The standards were also used for decay correction.

The percent injected dose per gram, noted as %ID/g, was calculated for each organ collected (mean ± standard deviation).

### * Results:

The results are illustrated in figure 5.

As shown in this figure, the ²¹²Pb-DOTAM-conjugate has a superior biodistribution profile than the ²¹²Pb-DOTA-conjugate with a higher tumor retention over the first 24 hours.

A higher initial uptake is observed in the pancreas at 1 hour post-dose injection for the ²¹²Pb-DOTAM-conjugate.

As previously mentioned, GRPR is known to be expressed in the pancreas and this initial higher uptake is likely a translation of the higher binding affinity of the ²¹²Pb-DOTAM-conjugate over the ²¹²Pb-DOTA-conjugate for cells expressing GRPR.

### IV.2 - Impact of a change of linker on the biodistribution in mice:

### Study in xenograft-bearing mice:

The instant study was aimed at assessing the biodistribution of a conjugate labelled with ²¹²Pb at a specific activity of 10 µCi per 10 ng and only differing from the conjugate of the invention in that it comprises a linker constituted by a chain of three glutamic acid residues, in athymic nude mice bearing a human prostate cancer cell tumor.

This conjugate is denoted hereinafter "²¹²Pb-3Glu-conjugate".

### * Preparation of the unlabelled 3Glu-conjugate:

The unlabelled 3Glu-conjugate was prepared following the same protocol as described under item I above except that the two β-alanine residues were replaced with three glutamic residues at the step of preparation of the peptide sequence.

### * ²¹²Pb-labelling of the 3Glu-conjugate at 10 µCi per 10 ng of conjugate:

| **Solutions** | **Volumes** | **Free ²¹²Pb** |
|---|---|---|
| 3Glu-conjugate (17 ng/µL) | 14.7 µL | <5% |
| ²¹²Pb (0.586 µCi/µL) | 427 µL | |

### * Preparation of ²¹²Pb-3Glu-conjugate doses of ≈ 10 µCi/100 µL:

| **Solutions** | **Volumes** |
|---|---|
| ²¹²Pb-3Glu-conjugate (0.415 µCi/µL) | 203.6 µL |
| Saline | 546.4 µL |

Insulin syringes each containing 100 µL of the solution resulting from the mixture ²¹²Pb-3Glu-conjugate/saline and corresponding to one ²¹²Pb-3Glu-conjugate dose of ≈ 10 µCi/100 µL were prepared for injection to mice.

### * Study design:

5 male athymic nude mice, 7-8 weeks old and weighting 21.25 ± 0.9 g at study initiation, were injected subcutaneously, into the right flank, with 10⁶ PC-3 human prostate cancer cells in 100 µL of RPMI-1640 medium/Matrigel^{™} (v/v: 1/1). The tumors were let grow until they reached 200-300 mm³.

Each mouse received intravenously one ²¹²Pb-3Glu-conjugate dose.

The mice were sacrificed at 4 hour post-dose injection.

Blood, bladder, reproductive organs, small intestine, colon with caecum, spleen, pancreas, kidneys, stomach, liver, lung, heart, brain, femoral bone, abdominal fat, skeletal muscle, tail and PC-3 tumor were collected from each sacrificed mouse, weighted and transferred to individual tubes for automatic gamma counter.

The tubes were counted for two min. Standards consisting of 5 µL of the solutions injected to the mice were also counted. The background was automatically subtracted from the counts. The standards were also used for decay correction.

The percent injected dose per gram, noted as %ID/g, was calculated for each organ collected (mean ± standard deviation).

### * Results:

The results are illustrated in figure 6.

As shown in this figure, simply replacing the -β-Ala-β-Ala- linker with a -Glu-Glu-Glu- linker results in a completely different biodistribution profile since no significant initial uptake in pancreas and no significant tumor uptake are observed for the ²¹²Pb-3Glu-conjugate.

### Study in tumor-free immunocompetent mice:

The study was aimed at assessing the biodistribution of a conjugate labelled with ²¹²Pb at a specific activity of 10 mCi per 4.1 ng and only differing from the conjugate of the invention in that it comprises a linker constituted by a 4-amino-(1-carboxymethyl)piperidinyl group, of formula: 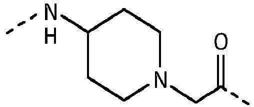 where the dotted lines represent the covalent bonds to the DOTAM and to the GRPR antagonist respectively, in tumor-free immunocompetent mice.

This conjugate is denoted hereinafter " ²¹²Pb-ACMP-conjugate".

### * Preparation of the unlabelled ACMP-conjugate:

The unlabelled ACMP-conjugate following the same protocol as described under item I above except that the peptide synthesis was stopped after the coupling of the DPhe and 4-amino-(1carboxymethyl)piperidine was conjugated to the peptide sequence before conjugating the DOTAM.

### * ²¹²Pb-labelling of the ACMP-conjugate at 10 µCi per 4.1 ng of conjugate:

*** Chelation took 30 min**

| **Solutions** | **Volumes** | **Free ²¹²Pb** |
|---|---|---|
| ACMP-conjugate (17 ng/µL) | 12.05 µL | <5%* |
| ²¹²Pb (1.07 µCi/µL) | 467 µL | |

### * Preparation of ²¹² Pb-ACMP-conjugate doses of ≈ 10 µCi/100 µL:

| **Solutions** | **Volumes** |
|---|---|
| ²¹²Pb-ACMP-conjugate (0.49 µCi/µL) | 177.6 µL |
| Saline | 572.4 µL |

Insulin syringes each containing 100 µL of the solution resulting from the mixture ²¹²Pb-ACMP-conjugate/saline and corresponding to one ²¹²Pb-ACMP-conjugate dose of ≈ 10 µCi/100 µL were prepared for injection to mice.

### * Study design:

5 immunocompetent CD1 female mice, 7-8 weeks old, were injected intravenously with one ²¹²Pb-ACMP-conjugate dose.

The mice were sacrificed at 4 hour post-dose injection.

Blood, bladder, reproductive organs, small intestine, colon with caecum, spleen, pancreas, kidneys, stomach, liver, lung, heart, brain, femoral bone, abdominal fat, skeletal muscle were collected from each sacrificed mouse, weighted and transferred to individual tubes for automatic gamma counter.

The tubes were counted for two min. Standards consisting of 5 µL of the solutions injected to the mice were also counted. The background was automatically subtracted from the counts. The standards were also used for decay correction.

The percent injected dose per gram, noted as %ID/g, was calculated for each organ collected (mean ± standard deviation).

### * Results:

The results are illustrated in figure 7.

As shown in this figure, simply replacing the -β-Ala-β-Ala- linker with a 4-amino-(1-carboxymethyl)piperidinyl linker results in a significantly lower safety profile with an uptake in kidneys which is 5 times higher.

### Reference cited

EP-A-2 252 628

## Claims

1. Conjugate or pharmaceutically acceptable salt thereof, the conjugate being of formula: C-L-A, wherein C is a chelator, L is a linker covalently bound to the chelator and A is a Gastrin-releasing peptide receptor antagonist covalently bound to the linker, **characterized in that**:
- the chelator is of formula: 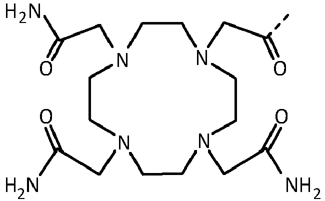 where the dotted line represents the covalent bond to the linker;
- the linker is of formula: -β-Ala-β-Ala-; and
- the Gastrin-releasing peptide receptor antagonist has the amino acid sequence: -DPhe-Gln-Trp-Ala-Val-Gly-His-Sta-Leu-NH₂ (SEQ ID NO: 1).

2. Conjugate or salt according to claim 1, which further comprises a radionuclide chelated by the chelator.

3. Conjugate or salt according to claim 2, in which the radionuclide is a lead radionuclide, preferably ²⁰³Pb or ²¹²Pb.

4. Composition, **characterized in that** it comprises a conjugate or salt according to claim 1, in a pharmaceutically acceptable medium.

5. Radiopharmaceutical, **characterized in that** it comprises a conjugate or salt according to claim 2 or claim 3, in a pharmaceutically acceptable medium.

6. Kit-of-parts, **characterized in that** it comprises at least:
- a first container containing a conjugate or salt according to claim 1; and
- a second container containing a radionuclide.

7. Kit-of-parts according to claim 6, in which the radionuclide is a lead radionuclide, preferably ²⁰³Pb or ²¹²Pb.

8. Use of a conjugate or salt according to claim 1, for preparing a radiopharmaceutical, comprising a chelation of a radionuclide by the chelator of the conjugate or salt thereof.

9. Use of a kit-of-parts according to claim 6 or claim 7, for preparing a radiopharmaceutical, comprising a chelation of the radionuclide by the chelator of the conjugate or salt thereof.

10. Use according to claim 8 or claim 9, in which the radionuclide is a lead radionuclide, preferably ²⁰³Pb or ²¹²Pb.

11. Radiopharmaceutical according to claim 5 for use in the *in vivo* imaging or the treatment of a cancer in which the Gastrin-releasing peptide receptor is overexpressed.

12. Radiopharmaceutical for the use of claim 11, in which the cancer is a prostate, breast or lung cancer, preferably a prostate cancer.


SEQUENCE LISTING

<110>  Orano Med.

<120>  CONJUGATE OR ITS SALT COMPRISING A GASTRIN-RELEASING PEPTIDE
       RECEPTOR ANTAGONIST AND USES THEREOF

<130>  S71853 EP

<140>  EP20XXXXX
<141>  2020-09-28

<160>  2

<170>  PatentIn version 3.5

<210>  1
<211>  9
<212>  PRT
<213>  Artificial Sequence

<220>
<223>  Gastrin-releasing peptide receptor (GRPR) antagonist  peptide
       known as JMV 594


<220>
<221>  MISC_FEATURE
<222>  (1)..(1)
<223>  D-Phe

<220>
<221>  MISC_FEATURE
<222>  (8)..(8)
<223>  Statine

<220>
<221>  MISC_FEATURE
<222>  (9)..(9)
<223>  Leu-NH2

<400>  1

Phe Gln Tyr Ala Val Gly His Xaa Leu
1               5


<210>  2
<211>  11
<212>  PRT
<213>  Artificial Sequence

<220>
<223>  Synthetic peptide


<220>
<221>  MISC_FEATURE
<222>  (1)..(1)
<223>  Beta-Ala

<220>
<221>  MISC_FEATURE

<222>  (2)..(2)
<223>  Beta-Ala

<220>
<221>  MISC_FEATURE
<222>  (3)..(3)
<223>  D-Phe

<220>
<221>  MISC_FEATURE
<222>  (10)..(10)
<223>  Statine

<220>
<221>  MISC_FEATURE
<222>  (11)..(11)
<223>  Leu-NH2

<400>  2

Ala Ala Phe Gln Tyr Ala Val Gly His Xaa Leu
1               5                   10